# FASCICULE DE BREVET EUROPEEN

(11) **EP 4 088 657 B1**
(45) Date de publication et mention de la délivrance du brevet: **18.09.2024**
(21) Numéro de dépôt: 22173398.3
(22) Date de dépôt: 13.05.2022
(51) Int. Cl.: A61B 5/12, A61B 5/11, G16H 40/63, G16H 50/30, G16H 50/50, A61B 5/00

(54) **SYSTÈMES ET PROCÉDÉS POUR DÉTERMINER UN SCORE DE LOCALISATION AUDITIVE SPATIALE**
SYSTEME UND VERFAHREN ZUR BESTIMMUNG EINER BEWERTUNG FÜR DIE RÄUMLICHE LOKALISATION DES GEHÖRS
SYSTEMS AND METHODS FOR DETERMINING A SCORE FOR SPATIAL LOCALIZATION HEARING

(30) Priorité: 13.05.2021 FR 2105070
(43) Date de publication de la demande: 16.11.2022
(73) Titulaire: Calyxen, 46240 Montfaucon (FR)
(72) Inventeur: DEFRANCE, Romaric, 46240 MONTFAUCON (FR); POTIER, Morgan, 11100 Montredon-Des-Corbières (FR); REMBAUD, Frédéric, 45430 CHECY (FR)
(74) Mandataire: A.P.I. Conseil

(56) Documents cités:
- WO-A1-2020/212404
- US-A1- 2014 257 131
- US-A1- 2020 387 341
- WOLF MARIO ET AL: "Implementing Continuous-Azimuth Binaural Sound in Unity 3D", 2020 IEEE CONFERENCE ON VIRTUAL REALITY AND 3D USER INTERFACES ABSTRACTS AND WORKSHOPS (VRW), IEEE, 22 March 2020 (2020-03-22), pages 384 - 389, XP033770064, DOI: 10.1109/VRW50115.2020.00083
- VALZOLGHER CHIARA ET AL: "Reaching to sounds in virtual reality: A multisensory-motor approach to promote adaptation to altered auditory cues", NEUROPSYCHOLOGIA, PERGAMON PRESS, OXFORD, GB, vol. 149, 29 October 2020 (2020-10-29), XP086395333, ISSN: 0028-3932, [retrieved on 20201029], DOI: 10.1016/J.NEUROPSYCHOLOGIA.2020.107665

## Description

### Domaine technique

L'invention concerne le domaine de l'audiométrie. En particulier, il concerne un système et un procédé pour déterminer un score de localisation auditive spatiale.

Des systèmes et procédés similaires sont divulgués dans le document US2020387341A1.

### Technique antérieure

L'évaluation audiométrique est l'un des éléments principaux de l'orientation diagnostique et thérapeutique face aux troubles de l'audition, tels que l'hypoacousie, les acouphènes et/ou l'hyperacousie.

En effet, les renseignements fournis par l'audiométrie traditionnellement pratiquée contribuent à établir un diagnostic otologique : siège probable de la lésion, pronostic, possibilités thérapeutiques et résultats fonctionnels obtenus.

On sait que l'évaluation audiométrique clinique en cabine est habituellement réalisée dans un environnement de test peu représentatif de l'expérience auditive quotidienne du patient.

Or, il serait souhaitable de réaliser des évaluations audiométriques dans des espaces sonores naturels ou artificiels.

Malheureusement, de tels espaces sont peu contrôlables et reproductibles, notamment au niveau du bruit qui peut y régner.

Ainsi, il existe un besoin pour réaliser des évaluations audiométriques dans des espaces sonores naturels ou artificiels, et ce, de manière contrôlable et reproductible.

### Résumé de l'invention

L'invention vise à résoudre, au moins partiellement, ce besoin.

L'invention vise en particulier un système pour déterminer un score représentatif de la capacité de localisation auditive spatiale d'un patient, appelé ci-après localisation auditive spatiale.

En particulier, le système comprend :
- un dispositif de sortie visuelle configuré pour être porté au niveau de la tête du patient de sorte qu'un mouvement de la tête du patient dans une direction de mouvement provoque un mouvement simultané et proportionnel du dispositif de sortie visuelle dans la direction de mouvement, le dispositif de sortie visuelle comprenant au moins une unité d'affichage destinée à afficher un environnement virtuel en direction d'au moins un oeil du patient,
- un dispositif de sortie sonore configuré pour diffuser au moins un signal audio de sortie au niveau d'au moins une oreille du patient,
- un dispositif de détection de mouvement configuré pour détecter au moins un mouvement de tout ou partie du corps d'un patient et générer au moins une mesure représentative d'un mouvement du patient,
- au moins un processeur couplé au dispositif de sortie visuelle, au dispositif de sortie sonore et au dispositif de détection de mouvement

Par ailleurs, le processeur est configuré pour :
- obtenir un espace auditif virtuel associé à l'environnement virtuel, à partir d'un modèle représentatif du comportement acoustique d'au moins un espace auditif physique du monde réel ou d'un monde artificiel, l'espace auditif virtuel étant configuré pour reproduire, dans l'environnement virtuel, le comportement acoustique de l'espace auditif physique du monde réel ou d'un monde artificiel,
- insérer et disposer, dans l'espace auditif virtuel, une pluralité de positions virtuelles de sources sonores virtuelles, autour d'une position virtuelle d'observation de l'environnement virtuel par le patient,
- commander le dispositif de sortie sonore pour diffuser le signal audio de sortie de sorte que, pour le patient, le signal audio de sortie semble provenir d'au moins l'une des positions virtuelles de sources sonores virtuelles,
- détecter au moins un premier mouvement du patient ou induit par le patient, en réponse à la diffusion du signal audio de sortie,
- obtenir, de la part du dispositif de détection de mouvement, au moins une mesure, en réponse à la détection d'au moins un premier mouvement du patient ou induit par le patient et jusqu'à la détection d'au moins un deuxième mouvement prédéterminé du patient ou induit par le patient, et
- calculer au moins un score de localisation auditive spatiale du patient, au moins à partir de l'espace auditif virtuel et la mesure, le score de localisation auditive étant représentatif de la précision de la prise de décision par le patient dans sa recherche de la source sonore virtuelle dont provient le signal audio de sortie, et comprenant le calcul d'un paramètre, X3, qui détermine la manière dont le patient déplace tout ou partie de son corps à la recherche de la source sonore virtuelle dont provient le signal audio de sortie.

Dans un premier mode de réalisation, le paramètre est un compteur, et le processeur est configuré pour :
- maintenir la valeur du compteur à une valeur prédéterminée tandis que le patient s'oriente vers une direction prédéterminée, et
- faire varier la valeur du compteur en fonction d'au moins changement de direction différente de la direction prédéterminée.

Dans un deuxième mode de réalisation, le processeur est en outre configuré pour:
- obtenir un modèle biomathématique qui décrit le score de localisation auditive spatiale en fonction d'au moins une classe de capacité de localisation auditive, et
- utiliser le score de localisation auditive spatiale du patient en tant qu'entrée du modèle biomathématique de manière à déterminer la classe de capacité de localisation auditive à laquelle appartient le patient.

Dans un deuxième du premier mode de réalisation, le modèle biomathématique décrit le score de localisation auditive, en outre, en fonction d'au moins une caractéristique auditive physiologique propre à la morphologie d'un auditeur, le processeur étant en outre configuré pour :
- obtenir au moins une caractéristique auditive physiologique du patient, et
- utiliser le score de localisation auditive spatiale du patient et la caractéristique auditive physiologique du patient en tant qu'entrées du modèle biomathématique de manière à déterminer la classe de capacité de localisation auditive à laquelle appartient le patient.

Dans un troisième mode de réalisation, le système comprend en outre une manette de commande mobile, dont la position et l'orientation sont déterminées par le patient, et qui est couplée au processeur. Par ailleurs, le dispositif de détection de mouvement comprend au moins un deuxième capteur d'orientation intégré à la manette de commande, le deuxième capteur d'orientation étant configuré pour fournir au moins une mesure représentative de l'orientation dans l'espace de la manette de commande.

En particulier, dans le troisième mode de réalisation, la manette de commande comprend en outre au moins un bouton de commande, le bouton de commande étant configuré pour générer un signal indicatif de la détection du deuxième mouvement prédéterminé.

L'invention vise aussi un procédé pour déterminer un score représentatif de la capacité de localisation auditive spatiale d'un patient, appelé ci-après localisation auditive spatiale.

En particulier, le procédé comprend :
- une première étape de fourniture d'un dispositif de sortie visuelle configuré pour être porté au niveau de la tête du patient de sorte qu'un mouvement de la tête du patient dans une direction de mouvement provoque un mouvement simultané et proportionnel du dispositif de sortie visuelle dans la direction de mouvement, le dispositif de sortie visuelle comprenant au moins une unité d'affichage destinée à afficher un environnement virtuel en direction d'au moins un oeil du patient,
- une deuxième étape de fourniture d'un dispositif de sortie sonore configuré pour diffuser au moins un signal audio de sortie au niveau d'au moins une oreille du patient,
- une troisième étape de fourniture d'au moins un mouvement de tout ou partie du corps d'un patient et générer au moins une mesure représentative d'un mouvement prédéterminé du patient,
- une quatrième étape de fourniture d'au moins un processeur couplé au dispositif de sortie visuelle, au dispositif de sortie sonore et au dispositif de détection de mouvement,
- une première étape d'obtention, par le processeur, d'un espace auditif virtuel associé à l'environnement virtuel, à partir d'un modèle représentatif du comportement acoustique d'au moins un espace auditif physique du monde réel ou d'un monde artificiel, de sorte que l'espace auditif virtuel reproduit, dans l'environnement virtuel, le comportement acoustique de l'espace auditif physique du monde réel ou d'un monde artificiel,
- une étape d'insertion et de disposition, dans l'espace auditif virtuel, par le processeur, d'une pluralité de positions virtuelles de sources sonores virtuelles, autour d'une position virtuelle d'observation de l'environnement virtuel par le patient,
- une étape de commande, par le processeur, du dispositif de sortie sonore pour diffuser le signal audio de sortie de sorte que, pour le patient, le signal audio de sortie semble provenir d'au moins l'une des positions virtuelles de sources sonores virtuelles,
- une étape de détection, par le processeur, d'au moins un premier mouvement du patient, en réponse à la diffusion du signal audio de sortie,
- une deuxième étape d'obtention, de la part du dispositif de détection de mouvement, par le processeur, d'au moins une mesure, en réponse à la détection d'au moins un premier mouvement du patient ou induit par le patient et jusqu'à la détection d'au moins un deuxième mouvement prédéterminé du patient ou induit par le patient, et
- une étape de calcul, par le processeur, d'au moins un score de localisation auditive spatiale du patient, au moins à partir de l'espace auditif virtuel et la mesure, le score de localisation auditive étant représentatif de la précision de la prise de décision par le patient dans sa recherche de la source sonore virtuelle dont provient le signal audio de sortie, et comprenant le calcul d'un paramètre qui détermine la manière dont le patient déplace tout ou partie de son corps à la recherche de la source sonore virtuelle dont provient le signal audio de sortie.

Dans un premier mode de réalisation, le paramètre est un compteur,
le procédé comprenant en outre :
- une étape de maintien, par le processeur, de la valeur du compteur à une valeur prédéterminée tandis que le patient s'oriente vers une direction prédéterminée, et
- une étape de variation, par le processeur, de la valeur du compteur en fonction d'au moins changement de direction différente de la direction prédéterminée.

Dans un deuxième mode de réalisation, le procédé comprend en outre :
- une troisième étape d'obtention, par le processeur, d'un modèle biomathématique, qui décrit le score de localisation auditive spatiale en fonction d'au moins une classe de capacité de localisation auditive, et
- une première étape d'utilisation, par le processeur, du score de localisation auditive spatiale du patient en tant qu'entrée du modèle biomathématique de manière à déterminer la classe de capacité de localisation auditive à laquelle appartient le patient.

Dans un troisième mode de réalisation, le modèle biomathématique décrit le score de localisation auditive, en outre, en fonction d'au moins une caractéristique auditive physiologique propre à la morphologie d'un auditeur, le procédé comprenant en outre :
- une quatrième étape d'obtention, par le processeur, d'au moins une caractéristique auditive physiologique du patient, et
- une deuxième étape d'utilisation, par le processeur, du score de localisation auditive spatiale du patient et la caractéristique auditive physiologique du patient en tant qu'entrées du modèle biomathématique de manière à déterminer la classe de capacité de localisation auditive à laquelle appartient le patient.

Dans un quatrième mode de réalisation, le procédé comprend en outre,
- une cinquième étape de fourniture d'une manette de commande (160) mobile, dont la position et l'orientation sont déterminées par le patient, et qui est couplée au processeur,
- une sixième étape de fourniture, au dispositif de détection de mouvement, d'au moins un deuxième capteur d'orientation intégré à la manette de commande, le deuxième capteur d'orientation étant configuré pour fournir au moins une mesure représentative de l'orientation dans l'espace de la manette de commande, et
- une septième étape de fourniture, à la manette de commande, au moins un bouton de commande, le bouton de commande étant configuré pour générer un signal indicatif de la détection du deuxième mouvement prédéterminé.

### Brève description des dessins

D'autres caractéristiques et avantages de l'invention seront mieux compris à la lecture de la description qui va suivre et en référence aux dessins annexés, donnés à titre illustratif et nullement limitatif.
[Fig. 1] La figure 1 représente un mode de réalisation du système selon l'invention.
[Fig. 2] La figure 2 représente un mode de réalisation d'un procédé selon l'invention.

Les figures ne respectent pas nécessairement les échelles, notamment en épaisseur, et ce à des fins d'illustration.

Sur les différentes figures, les traits et flèches en pointillés indiquent des éléments, des étapes et des enchaînements facultatifs ou optionnels.

### Description des modes de réalisation

L'un des objectifs de cette invention est de permettre la réalisation d'évaluations audiométriques dans des espaces sonores naturels ou artificiels, et ce, de manière contrôlable et reproductible.

Pour cela, les inventeurs proposent de créer des environnements virtuels qui reproduisent des caractéristiques sonores et visuelles d'espaces naturels ou artificiels. En pratique, on initie une expérience utilisateur entre un sujet et un environnement virtuel de manière à simuler un test d'audiométrie spécifique. Enfin, on détermine un score de localisation auditive spatiale à partir de mesures que l'on aura réalisées dans l'environnement virtuel.

Avec un tel agencement, les inventeurs ont confirmé la possibilité de réaliser des évaluations audiométriques de manière contrôlable et reproductible.

Ainsi, l'invention se rapporte à un système pour déterminer un score représentatif de la capacité de localisation auditive spatiale d'un patient, appelé ci-après score de localisation auditive spatiale.

De manière classique, on peut utiliser le système selon l'invention pour évaluer la capacité de localisation auditive spatiale d'un patient, à savoir, la localisation dans le plan horizontal (dite localisation azimutale), la localisation dans le plan vertical (dite localisation en élévation) et/ou la localisation en distance (c.-à-d. la distance entre la source sonore et le patient).

En outre, on peut également utiliser le système selon l'invention dans le cadre d'un entraînement pour l'amélioration de la capacité de localisation auditive spatiale d'un patient.

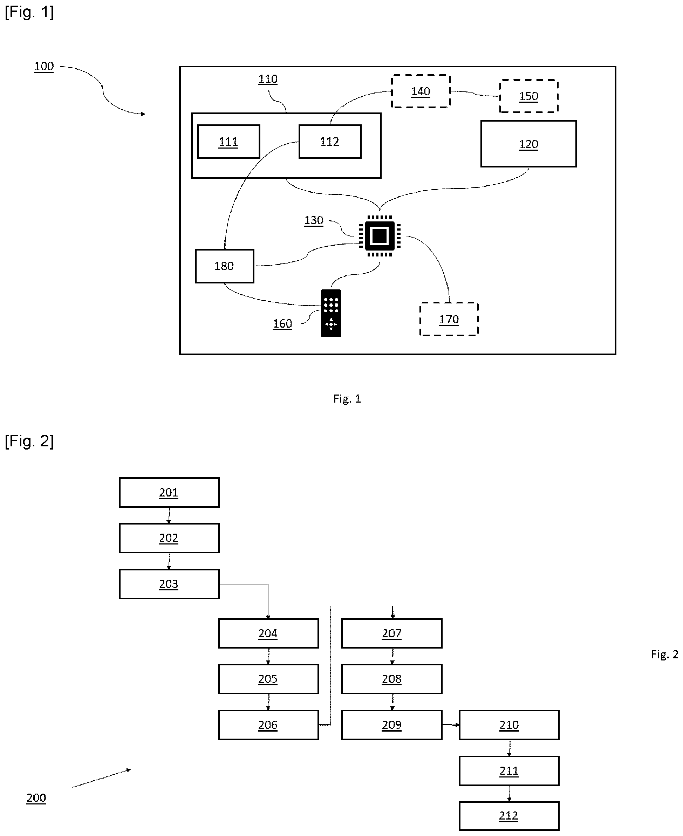

Comme illustré dans l'exemple de la figure 1, le système 100 comprend un dispositif de sortie visuelle 110, un dispositif de sortie sonore 120, un dispositif de détection de mouvement 180 et au moins un processeur 130.

Le dispositif de sortie visuelle 110 est configuré pour être porté au niveau de la tête d'un patient.

Dans un exemple, le dispositif de sortie visuelle 110 comprend une enveloppe en forme de casque qui coiffe la tête du patient.

En particulier, le dispositif de sortie visuelle 110 est prévu pour être porté de sorte qu'un mouvement de la tête du patient dans une direction de mouvement provoque un mouvement simultané et proportionnel du dispositif de sortie visuelle 110 dans la direction de mouvement.

Par ailleurs, le dispositif de sortie visuelle 110 comprend au moins une unité d'affichage 111.

En particulier, l'unité d'affichage 111 est destinée à afficher un environnement virtuel en direction d'au moins un oeil du patient.

Par exemple, l'unité d'affiche 111 est un écran de visualisation.

Dans un premier exemple particulier, l'unité d'affichage 111 est destinée à afficher l'environnement virtuel en direction d'un seul oeil du patient.

Dans un deuxième exemple particulier, l'unité d'affichage 111 est destinée à afficher l'environnement virtuel en direction de chacun des yeux du patient.

Toutefois, selon les besoins, on pourra envisager d'autres agencements de l'unité d'affichage 111, et ce, sans nécessiter de modifications substantielles de l'invention.

Dans une mise en oeuvre particulière, l'unité d'affichage 111 est configurée pour permettre une interaction entre le patient et l'environnement virtuel.

Dans un exemple, l'environnement virtuel est choisi parmi : un mode de réalité augmentée, un mode de réalité virtuelle et une combinaison de ceux-ci.

Dans un mode de réalisation particulier, le système 100 comprend en outre, une unité de génération d'environnement virtuel 140 couplée au dispositif de sortie visuelle 110.

En pratique, l'unité de génération d'environnement virtuel 140 est du type connu et est configurée pour générer au moins une image d'un environnement virtuel.

Dans un exemple du mode de réalisation particulier, le système 100 comprend aussi une unité d'acquisition d'images 150 que l'on couple à l'unité de génération d'environnement virtuel 140.

En pratique, l'unité d'acquisition d'images 150 est du type connu et est configurée pour acquérir au moins une image d'un environnement réel.

Par ailleurs, l'unité de génération d'environnement virtuel 140 est en outre configurée pour générer l'image de l'environnement virtuel à partir de l'image de l'environnement réel.

Toujours dans la figure 1, le dispositif de sortie sonore 120 est configuré pour diffuser au moins un signal audio de sortie au niveau d'au moins une oreille du patient.

Dans un exemple, le dispositif de sortie sonore 120 est un transducteur audio, tel que choisi parmi : un casque d'écoute, un système d'écouteur et une combinaison de ceux-ci.

Dans l'exemple de la figure 1, le dispositif de détection de mouvement 180 est configuré pour détecter au moins un mouvement de tout ou partie du corps d'un patient et générer au moins une mesure représentative d'un mouvement du patient.

Dans une première mise en oeuvre particulière, le dispositif de détection de mouvement 180 comprend au moins un premier capteur d'orientation 112 qui est intégré au dispositif de sortie visuelle 110. En particulier, le premier capteur d'orientation 112 est configuré pour fournir au moins une mesure qui est représentative de l'orientation dans l'espace du dispositif de sortie visuelle 110.

Dans un exemple, le premier capteur d'orientation 112 est choisi parmi : une unité de mesure inertielle, un gyroscope, un gyroscope triaxial, un accéléromètre, accéléromètre triaxial et une combinaison de ceux-ci.

Dans une deuxième mise en oeuvre particulière, le dispositif de détection de mouvement 180 comprend au moins une unité de détection de mouvement des yeux qui est intégrée au dispositif de sortie visuelle 110. En particulier, l'unité de détection de mouvement des yeux est configurée pour fournir au moins une mesure qui est représentative du mouvement des yeux du patient.

Dans un exemple, l'unité de détection de mouvement des yeux comprend un ensemble de miroirs et de capteurs infrarouges.

Dans une troisième mise en oeuvre particulière, le système 100 comprend une manette de commande 160 qui est mobile, dont la position et l'orientation sont déterminées par le patient, et qui est couplée au processeur 130. Par ailleurs, le dispositif de détection de mouvement 180 comprend au moins un deuxième capteur d'orientation qui est intégré à la manette de commande 160. En particulier, le deuxième capteur d'orientation est configuré pour fournir au moins une mesure qui est représentative de l'orientation dans l'espace de la manette de commande 160.

Dans un exemple, le deuxième capteur d'orientation est choisi parmi : une unité de mesure inertielle, un gyroscope, un gyroscope triaxial, un accéléromètre, accéléromètre triaxial et une combinaison de ceux-ci.

Dans une quatrième mise en oeuvre particulière, le dispositif de détection de mouvement 180 comprend au moins une unité de traitement d'image 170 qui est couplée au dispositif de sortie visuelle 110. En particulier, l'unité de traitement d'image 170 est configurée pour fournir au moins une mesure qui est représentative d'un mouvement de main du patient qui interagit avec l'environnement virtuel.

De retour à la figure 1, le processeur 130 est couplé au dispositif de sortie visuelle 110, au dispositif de sortie sonore 120 et au dispositif de détection de mouvement 180.

Tout d'abord, le processeur 130 est configuré pour obtenir un espace auditif virtuel qui est associé à l'environnement virtuel.

De manière connue, un espace auditif virtuel (« Virtual Auditory Space », VAS, en anglais) est une scène sonore virtuelle qui comprend un ensemble de sources sonores qui n'existent que dans l'espace perceptif d'un auditeur.

En d'autres termes, un espace auditif virtuel n'existe que dans la perception de l'auditeur et représente, à ce titre, une image mentale qui est suggérée à l'auditeur. De manière classique, on met en oeuvre une telle suggestion par le biais de signaux acoustiques qui sont appliqués aux tympans de l'auditeur et qui sont convenablement contrôlés de manière à produire l'illusion auditive souhaitée.

En pratique, on peut créer un espace auditif virtuel à partir d'un modèle représentatif du comportement acoustique d'au moins un espace auditif physique du monde réel ou d'un monde artificiel.

Ainsi, l'espace auditif virtuel peut être configuré pour reproduire, dans l'environnement virtuel, le comportement acoustique de l'espace auditif physique du monde réel ou d'un monde artificiel.

Dans un premier mode de réalisation particulier, l'espace auditif virtuel utilise un modèle de représentation d'une scène audio tridimensionnelle de type connu.

Par exemple, un tel modèle de représentation d'une scène audio tridimensionnelle peut être choisi parmi les technologies suivantes : stéréophonie, multicanal 5.1, ambisonique et ambisonique d'ordre supérieur (« Ambisonic and Higher Order Ambisonic », HOA, en anglais), holophonique (« Holophonie and Wave Field Synthesis », WFS, en anglais), binaurale, panoramisation d'amplitude ou d'intensité à base de vecteur (« Vector Base Amplitude Panning and Vectore Base Intensity Panning », VBAP and VBIP, en anglais) et une combinaison de celles-ci.

Dans un deuxième mode de réalisation particulier, l'espace auditif virtuel présente des caractéristiques de propagation de son virtuel.

Dans l'invention, le processeur 130 utilise les caractéristiques de propagation de son de l'espace auditif virtuel pour générer le signal audio de sortie.

En pratique, le processeur 130 diffuse un signal audio d'entrée dans l'espace auditif virtuel. Par la suite, le processeur 130 encode le signal audio d'entrée qui est diffusé de manière à former des données de son d'un type prédéterminé. Par exemple, le type prédéterminé peut être un type ambisonique d'un ordre R, avec R qui est un entier naturel supérieur à 1. Dans un tel exemple, le processeur 130 peut réaliser la transformation à l'aide d'une transformée de Fourier rapide, d'une multiplication matricielle, d'une transformée de Fourier rapide inverse ou à l'aide d'un filtre passe-bande. Enfin, le processeur 130 décode les données de son pour former le signal audio de sortie.

Dans un troisième mode de réalisation particulier, l'environnement virtuel comprend au moins une structure d'occlusion de son qui est configurée pour modifier, dans l'espace auditif virtuel, la propagation d'une onde sonore.

Par exemple, la structure d'occlusion de son peut modifier la propagation du signal audio d'entrée en utilisant des phénomènes physiques choisis parmi : la réflexion, la diffraction, la réfraction, l'absorption, la génération d'au moins une interférence et une combinaison de ceux-ci.

De retour à la figure 1, le processeur 130 est en outre configuré pour insérer et disposer, dans l'espace auditif virtuel, une pluralité de positions virtuelles de sources sonores virtuelles.

De préférence, le processeur 130 dispose la pluralité de positions virtuelles de sources sonores virtuelles autour d'une position virtuelle d'observation de l'environnement virtuel par le patient.

Dans une mise en oeuvre particulière, tout ou partie de la pluralité de positions virtuelles de sources sonores virtuelles est mobile dans l'environnement virtuel entre une position de départ respective et une position d'arrivée respective.

Par la suite, le processeur 130 est en outre configuré pour commander le dispositif de sortie sonore 120 pour diffuser le signal audio de sortie de sorte que, pour le patient, le signal audio de sortie semble provenir d'au moins l'une des positions virtuelles de sources sonores virtuelles.

Puis, le processeur 130 est en outre configuré pour détecter au moins un premier mouvement du patient ou induit par le patient, en réponse à la diffusion du signal audio de sortie.

Après, le processeur 130 est en outre configuré pour obtenir, de la part du dispositif de détection de mouvement 180, au moins une mesure, en réponse à la détection d'au moins un premier mouvement du patient ou induit par le patient et jusqu'à la détection d'au moins un deuxième mouvement prédéterminé du patient ou induit par le patient.

Dans un exemple, le processeur 130 obtient périodiquement, à une période prédéterminée, au moins une mesure. Par exemple, la période prédéterminée est comprise entre 100 ms et 1 seconde.

Dans une première mise en oeuvre particulière, lorsque le dispositif de détection de mouvement 180 comprend le premier capteur d'orientation 112 qui est intégré au dispositif de sortie visuelle 110, le premier mouvement et le deuxième mouvement prédéterminé peuvent être choisis parmi : un pivotement de la tête du patient par rapport à un axe prédéterminé, un mouvement de translation de la tête du patient selon un axe de translation et un mouvement de rotation de la tête du patient autour d'un axe de rotation.

Toutefois, selon les besoins, on pourra envisager d'autres mouvements de la tête, par exemple, pendant une période prédéterminée, et ce, sans nécessiter de modifications substantielles de l'invention.

Dans une deuxième mise en oeuvre particulière, lorsque le dispositif de détection de mouvement 180 comprend l'unité de détection de mouvement des yeux qui est intégrée au dispositif de sortie visuelle 110, le premier mouvement et le deuxième mouvement prédéterminé peuvent être choisis parmi : un mouvement d'inclinaison des yeux du patient par rapport à un axe prédéterminé, un mouvement de translation des yeux du patient selon un axe de translation et un mouvement de rotation des yeux du patient autour d'un axe de rotation.

Toutefois, selon les besoins, on pourra envisager d'autres mouvements des yeux, par exemple, pendant une période prédéterminée, et ce, sans nécessiter de modifications substantielles de l'invention.

Dans une troisième mise en oeuvre particulière, lorsque le système 100 comprend la manette de commande 160 qui intègre le deuxième capteur d'orientation, le premier mouvement et le deuxième mouvement prédéterminé peuvent être choisis parmi : un pivotement de la manette de commande 160 par rapport à un axe prédéterminé, un mouvement de translation de la manette de commande 160 selon un axe de translation et un mouvement de rotation de la manette de commande 160 autour d'un axe de rotation.

Toutefois, selon les besoins, on pourra envisager d'autres mouvements de la manette de commande 160, par exemple, pendant une période prédéterminée, et ce, sans nécessiter de modifications substantielles de l'invention.

Dans un mode de réalisation de la troisième mise en oeuvre particulière, la manette de commande 160 comprend en outre au moins un bouton de commande. En particulier, le bouton de commande est configuré pour générer un signal indicatif de la détection du deuxième mouvement prédéterminé.

Ainsi, avec cet agencement, l'appui sur le bouton de commande par le patient permet au processeur 130 d'arrêter d'obtenir la mesure de la part du dispositif de détection de mouvement 180.

Dans une quatrième mise en oeuvre particulière, lorsque le dispositif de détection de mouvement 180 comprend l'unité de traitement d'image 170 qui est couplée au dispositif de sortie visuelle 110, le premier mouvement et le deuxième mouvement prédéterminé peuvent être choisis parmi : un pivotement de la main du patient par rapport à un axe prédéterminé, un mouvement de translation de la main du patient selon un axe de translation et un mouvement de rotation de la main du patient autour d'un axe de rotation.

Toutefois, selon les besoins, on pourra envisager d'autres mouvements de la main ou d'une autre partie du corps, par exemple, pendant une période prédéterminée, et ce, sans nécessiter de modifications substantielles de l'invention.

Enfin, le processeur 130 est en outre configuré pour calculer au moins un score de localisation auditive spatiale du patient, au moins à partir de l'espace auditif virtuel et la mesure.

L'invention n'est pas limitée à une manière particulière de calculer un score à partir de l'espace auditif virtuel et la mesure.

Ainsi, selon les besoins, on pourra envisager d'autres méthodes de calcul du score de localisation auditive spatiale que celles présentées ci-dessous, et ce, sans nécessiter de modifications substantielles de l'invention.

Par exemple, on pourra calculer plusieurs scores de localisation auditive spatiale avec le même signal audio de sortie, mais avec des sources sonores virtuelles qui sont disposées virtuellement à différentes distances du patient. Dans ce cas, on obtiendra au moins un score par distance virtuelle.

Dans un premier exemple, un premier score de localisation auditive spatiale du patient, appelée ci-après Sk1, comprend le calcul d'un écart de position entre la position de la source sonore virtuelle dont provient le signal audio de sortie, appelé ci-après X1, et la position indiquée par le patient via le dispositif de détection de mouvement 180, appelée ci-après X2.

Dans l'invention, on peut déterminer Sk1 à partir des positions dans le plan horizontal (dite localisation azimutale) et/ou dans le plan vertical (dite localisation en élévation).

Ainsi, dans un exemple, si X1 = 10° et X2 = 25°, alors Sk1 = X1-X2 = -15°.

Dans un deuxième exemple, un deuxième score de localisation auditive spatiale du patient, appelée ci-après Sk2, comprend la détermination de la précision de la prise de décision par le patient.

En particulier, Sk2 permet de prendre en compte la manière dont le patient déplace tout ou partie de son corps dans le plan horizontal (dite localisation azimutale) et/ou dans le plan vertical (dite localisation en élévation) à la recherche de la source sonore virtuelle dont provient le signal audio de sortie, ci-après appelé X3.

En pratique, X3 peut être un compteur qui reste nul tant que le patient s'oriente vers une direction, puis qui s'incrémente, par exemple à chaque degré en azimut de visée modifié, et ce jusqu'à la détection d'un mouvement par le patient via le dispositif de détection de mouvement 180. Dans un exemple, on pourra incrémenter X3 d'un degré pour chaque degré en azimut de visée modifié.

Ainsi, dans un exemple, on aura X3 = 30°, si X1 = 90° et que le patient a d'abord tourné tout ou partie de son corps jusqu'à 100°, puis s'est ravisé pour retourner à 80°, et enfin encore se déplacer à 90°. Dans ce cas, le patient aura bougé de 20° (lors du passage de 100° à 80°), puis de 10° (lors du passage de 80° à 90°), soit 30° au total (=20°+10°).

Dans cet exemple si X3 = 30° et que le temps de réponse au signal audio de sortie, appelé ci-après Tr, est tel que Tr = 4.5 s, alors Sk2 = (360-X3)/Tr = (360-30)/4.5 = 73.33.

De préférence, on pourra combiner les différents scores de localisation auditive spatiale pour obtenir un score final de localisation auditive spatiale.

Dans une première mise en oeuvre particulière, le processeur 130 est en outre configuré pour :
- obtenir un modèle biomathématique qui décrit le score de localisation auditive spatiale en fonction d'au moins une classe de capacité de localisation auditive, et
- utiliser le score de localisation auditive spatiale du patient en tant qu'entrée du modèle biomathématique de manière à déterminer la classe de capacité de localisation auditive à laquelle appartient le patient.

Ainsi, avec cette première mise en oeuvre particulière, on peut comparer le score de localisation auditive spatiale du patient aux scores obtenus par les normo-entendants et/ou les malentendants.

Dans un premier exemple, la classe de capacité de localisation auditive est une classe qui décrit des caractéristiques tonales comme la classe audiométrique de déficience auditive telle que définie par la recommandation 02/1 bis du bureau international d'audiophonologie (BIAP). Dans cette recommandation, les différentes classes peuvent être choisies parmi : audition normale, surdité légère, surdité moyenne, surdité sévère, surdité profonde et surdité totale.

Dans un deuxième exemple, la classe de capacité de localisation auditive est une classe qui décrit des caractéristiques d'âge.

Dans un troisième exemple, la classe de capacité de localisation auditive est une classe qui décrit des caractéristiques de champ auditif qui se définit comme l'aire délimitée par le seuil d'audition et le seuil d'inconfort dans la zone des fréquences audibles.

Dans un quatrième exemple, la classe de capacité de localisation auditive est une classe qui décrit des caractéristiques vocales comme l'intelligibilité vocale.

Toutefois, selon les besoins, on pourra envisager d'autres classes physiologiques, et ce, sans nécessiter de modifications substantielles de l'invention.

Dans une deuxième mise en oeuvre particulière, le modèle biomathématique décrit le score de localisation auditive, en outre, en fonction d'au moins une caractéristique auditive physiologique propre à la morphologie d'un auditeur.

Par exemple, la caractéristique auditive physiologique comprend une fonction de transfert relative à la tête (« Head Related Transfer Function », HRTF, en anglais) ou une réponse impulsionnelle relative à la tête (« Head-Related Impulse Response », HRIR, en anglais).

Ainsi, dans cette deuxième mise en oeuvre particulière, le processeur 130 est en outre configuré pour :
- obtenir au moins une caractéristique auditive physiologique du patient, et
- utiliser le score de localisation auditive spatiale du patient et la caractéristique auditive physiologique du patient en tant qu'entrées du modèle biomathématique de manière à déterminer la classe de capacité de localisation auditive à laquelle appartient le patient.

Ainsi, avec cette mise en oeuvre particulière, on peut obtenir une prédiction plus précise, car on pourra comparer le score de localisation auditive spatiale du patient aux scores obtenus par les normo-entendants et/ou les malentendants qui sont physiologiquement proches du patient.

Dans une troisième mise en oeuvre de l'invention, le modèle biomathématique est un modèle d'apprentissage automatique que l'on a entraîné sur une pluralité de n-uplet (« tuples », en anglais) comprenant au moins une classe de capacité de localisation auditive et au moins un score de localisation auditive spatiale.

Dans un exemple de la troisième mise en oeuvre de l'invention, chaque tuple peut comprendre une caractéristique auditive physiologique d'un auditeur.

Dans un mode de réalisation particulier, le processeur 130 est en outre configuré pour discriminer la prise en compte de la mesure pour calculer le score de localisation auditive spatiale, notamment lorsque le système comprend au moins deux mises en oeuvre particulières du dispositif de détection de mouvement 180 telles que décrites plus haut.

Dans un premier exemple de ce mode de réalisation particulier, le processeur 130 désactive la prise en compte de la mesure qui provient d'au moins une mise en oeuvre particulière du dispositif de détection de mouvement 180 mesure et ne prend en compte que la mesure qui provient d'une seule mise en oeuvre du dispositif de détection de mouvement 180.

Dans un deuxième exemple de ce mode de réalisation particulier, le processeur 130 prend en compte une combinaison des mesures qui proviennent d'au moins deux mises en oeuvre particulières du dispositif de détection de mouvement 180.

Dans un exemple, le processeur 130 calcule cette combinaison comme une grandeur mathématique choisie parmi : une moyenne, une médiane, un mode, un minimum et un maximum.

Toutefois, selon les besoins, on pourra envisager d'autres grandeurs mathématiques, et ce, sans nécessiter de modifications substantielles de l'invention.

L'invention concerne aussi un procédé pour déterminer un score qui est représentatif de la capacité de localisation auditive spatiale d'un patient, appelé ci-après score de localisation auditive spatiale.

Tout d'abord, comme présenté en lien avec la figure 2, le procédé 200 comprend une première étape de fourniture 201 d'un dispositif de sortie visuelle 110 tel que décrit plus haut.

Ensuite, le procédé 200 comprend une deuxième étape de fourniture 202 d'un dispositif de sortie sonore 120 tel que décrit plus haut.

Après, le procédé 200 comprend une troisième étape de fourniture 203 d'au moins une mesure représentative d'un mouvement prédéterminé du patient.

Puis, le procédé 200 comprend une quatrième étape de fourniture 204 d'au moins un processeur 130 tel que décrit plus haut, de sorte que le processeur 130 est couplé au dispositif de sortie visuelle 110, au dispositif de sortie sonore 120 et au dispositif de détection de mouvement 180.

Par la suite, le procédé 200 comprend une première étape d'obtention 205, par le processeur 130, d'un espace auditif virtuel associé à l'environnement virtuel, à partir d'un modèle représentatif du comportement acoustique d'au moins un espace auditif physique du monde réel ou d'un monde artificiel, de sorte que l'espace auditif virtuel reproduise, dans l'environnement virtuel, le comportement acoustique de l'espace auditif physique du monde réel ou d'un monde artificiel.

Après, le procédé 200 comprend une étape d'insertion et de disposition 206, dans l'espace auditif virtuel, par le processeur 130, d'une pluralité de positions virtuelles de sources sonores virtuelles, autour d'une position virtuelle d'observation de l'environnement virtuel par le patient.

Puis, le procédé 200 comprend une étape de commande 207, par le processeur 130, du dispositif de sortie sonore 120 pour diffuser le signal audio de sortie de sorte que, pour le patient, le signal audio de sortie semble provenir d'au moins l'une des positions virtuelles de sources sonores virtuelles.

Par la suite, le procédé 200 comprend une étape de détection 208, par le processeur 130, d'au moins un premier mouvement du patient ou induit par le patient, en réponse à la diffusion du signal audio de sortie.

Ensuite, le procédé 200 comprend une deuxième étape d'obtention 209, de la part du dispositif de détection de mouvement 180, par le processeur 130, d'au moins une mesure, en réponse à la détection d'au moins un premier mouvement du patient ou induit par le patient et jusqu'à la détection d'au moins un deuxième mouvement prédéterminé du patient ou induit par le patient.

Enfin, le procédé 200 comprend une étape de calcul 210, par le processeur 130, d'au moins un score de localisation auditive spatiale du patient, au moins à partir de l'espace auditif virtuel et la mesure.

Dans une mise en oeuvre particulière, le procédé 200 comprend en outre :
- une troisième étape d'obtention 211, par le processeur 130, d'un modèle biomathématique, qui décrit le score de localisation auditive spatiale en fonction d'au moins une classe de capacité de localisation auditive, et
- une étape d'utilisation 212, par le processeur 130, du score de localisation auditive spatiale du patient en tant qu'entrée du modèle biomathématique de manière à déterminer la classe de capacité de localisation auditive à laquelle appartient le patient.

Nous avons décrit et illustré l'invention. Toutefois, l'invention ne se limite pas aux formes de réalisations que nous avons présentées. La présente invention est plutôt définie par les revendications ci-jointes.

## Revendications

1. Système (100) pour déterminer un score représentatif de la capacité de localisation auditive spatiale d'un patient, appelé ci-après localisation auditive spatiale, le système (100) comprenant :
- un dispositif de sortie visuelle (110) configuré pour être porté au niveau de la tête du patient de sorte qu'un mouvement de la tête du patient dans une direction de mouvement provoque un mouvement simultané et proportionnel du dispositif de sortie visuelle (110) dans la direction de mouvement, le dispositif de sortie visuelle (110) comprenant au moins une unité d'affichage (111) destinée à afficher un environnement virtuel en direction d'au moins un oeil du patient,
- un dispositif de sortie sonore (120) configuré pour diffuser au moins un signal audio de sortie au niveau d'au moins une oreille du patient,
- un dispositif de détection de mouvement (180) configuré pour détecter au moins un mouvement de tout ou partie du corps d'un patient et générer au moins une mesure représentative d'un mouvement du patient,
- au moins un processeur (130) couplé au dispositif de sortie visuelle (110), au dispositif de sortie sonore (120) et au dispositif de détection de mouvement (180), le processeur (130) étant configuré pour :
- obtenir un espace auditif virtuel associé à l'environnement virtuel, à partir d'un modèle représentatif du comportement acoustique d'au moins un espace auditif physique du monde réel ou d'un monde artificiel, l'espace auditif virtuel étant configuré pour reproduire, dans l'environnement virtuel, le comportement acoustique de l'espace auditif physique du monde réel ou d'un monde artificiel,
- insérer et disposer, dans l'espace auditif virtuel, une pluralité de positions virtuelles de sources sonores virtuelles, autour d'une position virtuelle d'observation de l'environnement virtuel par le patient,
- commander le dispositif de sortie sonore (120) pour diffuser le signal audio de sortie de sorte que, pour le patient, le signal audio de sortie semble provenir d'au moins l'une des positions virtuelles de sources sonores virtuelles,
- détecter au moins un premier mouvement du patient ou induit par le patient, en réponse à la diffusion du signal audio de sortie,
- obtenir, périodiquement, de la part du dispositif de détection de mouvement (180), au moins une mesure, en réponse à la détection du premier mouvement du patient ou induit par le patient et jusqu'à la détection d'au moins un deuxième mouvement prédéterminé du patient ou induit par le patient, et
- calculer au moins un score de localisation auditive spatiale du patient, au moins à partir de l'espace auditif virtuel et la mesure, le score de localisation auditive étant représentatif de la précision de la prise de décision par le patient dans sa recherche de la source sonore virtuelle dont provient le signal audio de sortie, et comprenant le calcul d'un paramètre, X3, qui détermine la manière dont le patient déplace tout ou partie de son corps à la recherche de la source sonore virtuelle dont provient le signal audio de sortie.

2. Système (100) selon la revendication 1 dans lequel le paramètre, X3, est un compteur, et le processeur (130) est configuré pour :
- maintenir la valeur du compteur à une valeur prédéterminée tandis que le patient s'oriente vers une direction prédéterminée, et
- faire varier la valeur du compteur en fonction d'au moins changement de direction différente de la direction prédéterminée.

3. Système (100) selon l'une quelconque des revendications 1 à 2, dans lequel le processeur (130) est en outre configuré pour :
- obtenir un modèle biomathématique qui décrit le score de localisation auditive spatiale en fonction d'au moins une classe de capacité de localisation auditive, et
- utiliser le score de localisation auditive spatiale du patient en tant qu'entrée du modèle biomathématique de manière à déterminer la classe de capacité de localisation auditive à laquelle appartient le patient.

4. Système (100) selon la revendication 3, dans lequel le modèle biomathématique décrit le score de localisation auditive, en outre, en fonction d'au moins une caractéristique auditive physiologique propre à la morphologie d'un auditeur,
le processeur (130) étant en outre configuré pour :
- obtenir au moins une caractéristique auditive physiologique du patient, et
- utiliser le score de localisation auditive spatiale du patient et la caractéristique auditive physiologique du patient en tant qu'entrées du modèle biomathématique de manière à déterminer la classe de capacité de localisation auditive à laquelle appartient le patient.

5. Système (100) selon l'une quelconque des revendications 1 à 4, comprenant en outre une manette de commande (160) mobile, dont la position et l'orientation sont déterminées par le patient, et qui est couplée au processeur (130), dans lequel le dispositif de détection de mouvement (180) comprend au moins un deuxième capteur d'orientation intégré à la manette de commande (160), le deuxième capteur d'orientation étant configuré pour fournir au moins une mesure représentative de l'orientation dans l'espace de la manette de commande (160),
dans lequel la manette de commande (160) comprend en outre au moins un bouton de commande, le bouton de commande étant configuré pour générer un signal indicatif de la détection du deuxième mouvement prédéterminé.

6. Procédé (200) pour déterminer un score représentatif de la capacité de localisation auditive spatiale d'un patient, appelé ci-après localisation auditive spatiale, le procédé (200) comprenant :
- une première étape de fourniture (201) d'un dispositif de sortie visuelle (110) configuré pour être porté au niveau de la tête du patient de sorte qu'un mouvement de la tête du patient dans une direction de mouvement provoque un mouvement simultané et proportionnel du dispositif de sortie visuelle (110) dans la direction de mouvement, le dispositif de sortie visuelle (110) comprenant au moins une unité d'affichage (111) destinée à afficher un environnement virtuel en direction d'au moins un oeil du patient,
- une deuxième étape de fourniture (202) d'un dispositif de sortie sonore (120) configuré pour diffuser au moins un signal audio de sortie au niveau d'au moins une oreille du patient,
- une troisième étape de fourniture (203) d'au moins une mesure représentative d'un mouvement prédéterminé du patient,
- une quatrième étape de fourniture (204) d'au moins un processeur (130) couplé au dispositif de sortie visuelle (110), au dispositif de sortie sonore (120) et au dispositif de détection de mouvement (180),
- une première étape d'obtention (205), par le processeur (130), d'un espace auditif virtuel associé à l'environnement virtuel, à partir d'un modèle représentatif du comportement acoustique d'au moins un espace auditif physique du monde réel ou d'un monde artificiel, de sorte que l'espace auditif virtuel reproduit, dans l'environnement virtuel, le comportement acoustique de l'espace auditif physique du monde réel ou d'un monde artificiel,
- une étape d'insertion et de disposition (206), dans l'espace auditif virtuel, par le processeur (130), d'une pluralité de positions virtuelles de sources sonores virtuelles, autour d'une position virtuelle d'observation de l'environnement virtuel par le patient,
- une étape de commande (207), par le processeur (130), du dispositif de sortie sonore (120) pour diffuser le signal audio de sortie de sorte que, pour le patient, le signal audio de sortie semble provenir d'au moins l'une des positions virtuelles de sources sonores virtuelles,
- une étape de détection (208), par le processeur (130), d'au moins un premier mouvement du patient, en réponse à la diffusion du signal audio de sortie,
- une deuxième étape d'obtention (209), périodique, de la part du dispositif de détection de mouvement (180), par le processeur (130), d'au moins une mesure, en réponse à la détection du premier mouvement du patient ou induit par le patient et jusqu'à la détection d'au moins un deuxième mouvement prédéterminé du patient ou induit par le patient, et
- une étape de calcul (210), par le processeur (130), d'au moins un score de localisation auditive spatiale du patient, au moins à partir de l'espace auditif virtuel et la mesure, le score de localisation auditive étant représentatif de la précision de la prise de décision par le patient dans sa recherche de la source sonore virtuelle dont provient le signal audio de sortie, et comprenant le calcul d'un paramètre, X3, qui détermine la manière dont le patient déplace tout ou partie de son corps à la recherche de la source sonore virtuelle dont provient le signal audio de sortie.

7. Procédé (200) selon la revendication 6, dans lequel le paramètre, X3, est un compteur, le procédé (200) comprenant en outre :
- une étape de maintien, par le processeur (130), de la valeur du compteur à une valeur prédéterminée tandis que le patient s'oriente vers une direction prédéterminée, et
- une étape de variation, par le processeur (130), de la valeur du compteur en fonction d'au moins changement de direction différente de la direction prédéterminée.

8. Procédé (200) selon l'une quelconque des revendications 6 à 7, comprenant en outre :
- une troisième étape d'obtention (211), par le processeur (130), d'un modèle biomathématique, qui décrit le score de localisation auditive spatiale en fonction d'au moins une classe de capacité de localisation auditive, et
- une première étape d'utilisation (212), par le processeur (130), du score de localisation auditive spatiale du patient en tant qu'entrée du modèle biomathématique de manière à déterminer la classe de capacité de localisation auditive à laquelle appartient le patient.

9. Procédé (200) selon la revendication 8, dans lequel le modèle biomathématique décrit le score de localisation auditive, en outre, en fonction d'au moins une caractéristique auditive physiologique propre à la morphologie d'un auditeur,
le procédé (200) comprenant en outre :
- une quatrième étape d'obtention, par le processeur (130), d'au moins une caractéristique auditive physiologique du patient, et
- une deuxième étape d'utilisation, par le processeur (130), du score de localisation auditive spatiale du patient et la caractéristique auditive physiologique du patient en tant qu'entrées du modèle biomathématique de manière à déterminer la classe de capacité de localisation auditive à laquelle appartient le patient.

10. Procédé (200) selon l'une quelconque des revendications 6 à 9, comprenant en outre :
- une cinquième étape de fourniture d'une manette de commande (160) mobile, dont la position et l'orientation sont déterminées par le patient, et qui est couplée au processeur (130),
- une sixième étape de fourniture, au dispositif de détection de mouvement (180), d'au moins un deuxième capteur d'orientation intégré à la manette de commande (160), le deuxième capteur d'orientation étant configuré pour fournir au moins une mesure représentative de l'orientation dans l'espace de la manette de commande (160), et
- une septième étape de fourniture, à la manette de commande (160), au moins un bouton de commande, le bouton de commande étant configuré pour générer un signal indicatif de la détection du deuxième mouvement prédéterminé.

## Patentansprüche

1. System (100) zum Bestimmen eines Scores, der das Vermögen der räumlichen auditorischen Lokalisation eines Patienten, im Folgenden räumliche auditorische Lokalisation genannt, darstellt, wobei das System (100) umfasst:
- eine Vorrichtung (110) zur visuellen Ausgabe, die dazu konfiguriert ist, auf Höhe des Kopfes des Patienten getragen zu werden, so dass eine Bewegung des Kopfes des Patienten in einer Bewegungsrichtung eine gleichzeitige und proportionale Bewegung der Vorrichtung (110) zur visuellen Ausgabe in der Bewegungsrichtung bewirkt, wobei die Vorrichtung (110) zur visuellen Ausgabe mindestens eine Anzeigeeinheit (111) umfasst, die dazu vorgesehen ist, eine virtuelle Umgebung in der Richtung mindestens eines Auges des Patienten zu zeigen,
- eine Tonausgabevorrichtung (120), die dazu konfiguriert ist, mindestens ein Audioausgabesignal auf Höhe mindestens eines Ohrs des Patienten zu senden,
- eine Bewegungserfassungsvorrichtung (180), die dazu konfiguriert ist, mindestens eine Bewegung des gesamten oder eines Teils des Körpers eines Patienten zu erfassen und mindestens einen Messwert, der eine Bewegung des Patienten darstellt, zu erzeugen,
- mindestens einen Prozessor (130), der an die Vorrichtung (110) zur visuellen Ausgabe, an die Tonausgabevorrichtung (120) und an die Bewegungserfassungsvorrichtung (180) gekoppelt ist, wobei der Prozessor (130) konfiguriert ist zum:
- Beziehen eines virtuellen Klangraums, der mit der virtuellen Umgebung assoziiert ist, aus einem Modell, das das akustische Verhalten mindestens eines physischen Klangraums der realen Welt oder einer künstlichen Welt darstellt, wobei der virtuelle Klangraum dazu konfiguriert ist, das akustische Verhalten des physischen Klangraums der realen Welt oder einer künstlichen Welt in der virtuellen Umgebung zu reproduzieren,
- Einfügen und Anordnen einer Vielzahl von virtuellen Positionen von virtuellen Tonquellen um eine virtuelle Position einer Beobachtung der virtuellen Umgebung durch den Patienten in dem virtuellen Klangraum,
- Befehligen der Tonausgabevorrichtung (120), das Audioausgabesignal zu senden, so dass das Audioausgabesignal für den Patienten von mindestens einer der virtuellen Positionen von virtuellen Tonquellen zu kommen scheint,
- Erfassen mindestens einer ersten Bewegung des Patienten oder von dem Patienten induzierten Bewegung als Reaktion auf das Senden des Audioausgabesignals,
- periodisches Beziehen mindestens eines Messwerts von der Bewegungserfassungsvorrichtung (180) als Reaktion auf die Erfassung der ersten Bewegung des Patienten oder von dem Patienten induzierten Bewegung und bis zur Erfassung mindestens einer zweiten vorbestimmten Bewegung des Patienten oder von dem Patienten induzierten Bewegung, und
- Berechnen mindestens eines Scores der räumlichen auditorischen Lokalisation des Patienten zumindest aus dem virtuellen Klangraum und dem Messwert, wobei der Score der auditorischen Lokalisation die Präzision der Entscheidungsfindung durch den Patienten bei seiner Suche nach der virtuellen Tonquelle, von der das Audioausgabesignal stammt, darstellt und die Berechnung eines Parameters, X3, umfasst, der die Art und Weise bestimmt, auf die der Patient seinen gesamten oder einen Teil seines Körpers bei der Suche nach der virtuellen Tonquelle, von der das Audioausgabesignal stammt, verlagert.

2. System (100) nach Anspruch 1, wobei der Parameter X3 ein Zähler ist und der Prozessor (130) konfiguriert ist zum:
- Halten des Werts des Zählers auf einem vorbestimmten Wert, während der Patient in eine vorbestimmte Richtung gewandt ist, und
- Variieren des Werts des Zählers in Abhängigkeit von mindestens einer Richtungsänderung, die sich von der vorbestimmten Richtung unterscheidet.

3. System (100) nach einem der Ansprüche 1 bis 2, wobei der Prozessor (130) weiterhin konfiguriert ist zum:
- Beziehen eines biomathematischen Modells, das den Score der räumlichen auditorischen Lokalisation in Abhängigkeit von mindestens einer Klasse eines Vermögens der auditorischen Lokalisation beschreibt, und
- Verwenden des Scores der räumlichen auditorischen Lokalisation des Patienten als Eingabe in das biomathematische Modell, um die Klasse des Vermögens der auditorischen Lokalisation, zu der der Patient gehört, zu bestimmen.

4. System (100) nach Anspruch 3, wobei das biomathematische Modell den Score der auditorischen Lokalisation weiterhin in Abhängigkeit von mindestens einem physiologischen auditorischen Merkmal, das der Morphologie eines Zuhörers eigen ist, beschreibt,
wobei der Prozessor (130) weiterhin konfiguriert ist zum:
- Beziehen mindestens eines physiologischen auditorischen Merkmals des Patienten und
- Verwenden des Scores der räumlichen auditorischen Lokalisation des Patienten und des physiologischen auditorischen Merkmals des Patienten als Eingaben in das biomathematische Modell, um die Klasse des Vermögens der auditorischen Lokalisation, zu der der Patient gehört, zu bestimmen.

5. System (100) nach einem der Ansprüche 1 bis 4, weiterhin umfassend einen beweglichen Steuerhebel (160), dessen Position und Ausrichtung von dem Patienten bestimmt werden und der an den Prozessor (130) gekoppelt ist, wobei die Bewegungserfassungsvorrichtung (180) mindestens einen zweiten Ausrichtungssensor umfasst, der in den Steuerhebel (160) integriert ist, wobei der zweite Ausrichtungssensor dazu konfiguriert ist, mindestens einen Messwert bereitzustellen, der die Ausrichtung in dem Raum des Steuerhebels (160) darstellt,
wobei der Steuerhebel (160) weiterhin mindestens einen Steuerknopf umfasst, wobei der Steuerknopf dazu konfiguriert ist, ein Signal zu erzeugen, das die Erfassung der zweiten vorbestimmten Bewegung angibt.

6. Verfahren (200) zum Bestimmen eines Scores, der das Vermögen der räumlichen auditorischen Lokalisation eines Patienten, im Folgenden räumliche auditorische Lokalisation genannt, darstellt, wobei das Verfahren (200) umfasst:
- einen ersten Schritt eines Bereitstellens (201) einer Vorrichtung (110) zur visuellen Ausgabe, die dazu konfiguriert ist, auf Höhe des Kopfes des Patienten getragen zu werden, so dass eine Bewegung des Kopfes des Patienten in einer Bewegungsrichtung eine gleichzeitige und proportionale Bewegung der Vorrichtung (110) zur visuellen Ausgabe in der Bewegungsrichtung bewirkt, wobei die Vorrichtung (110) zur visuellen Ausgabe mindestens eine Anzeigeeinheit (111) umfasst, die dazu vorgesehen ist, eine virtuelle Umgebung in der Richtung mindestens eines Auges des Patienten zu zeigen,
- einen zweiten Schritt eines Bereitstellens (202) einer Tonausgabevorrichtung (120), die dazu konfiguriert ist, mindestens ein Audioausgabesignal auf Höhe mindestens eines Ohrs des Patienten zu senden,
- einen dritten Schritt eines Bereitstellens (203) mindestens eines Messwerts, der eine vorbestimmte Bewegung des Patienten darstellt,
- einen vierten Schritt eines Bereitstellens (204) mindestens eines Prozessors (130), der an die Vorrichtung (110) zur visuellen Ausgabe, an die Tonausgabevorrichtung (120) und an die Bewegungserfassungsvorrichtung (180) gekoppelt ist,
- einen ersten Schritt eines Beziehens (205) eines virtuellen Klangraums, der mit der virtuellen Umgebung assoziiert ist, aus einem Modell, das das akustische Verhalten mindestens eines physischen Klangraums der realen Welt oder einer künstlichen Welt darstellt, durch den Prozessor (130), so dass der virtuelle Klangraum das akustische Verhalten des physischen Klangraums der realen Welt oder einer künstlichen Welt in der virtuellen Umgebung reproduziert,
- einen Schritt eines Einfügens und eines Anordnens (206) einer Vielzahl von virtuellen Positionen von virtuellen Tonquellen um eine virtuelle Position einer Beobachtung der virtuellen Umgebung durch den Patienten in dem virtuellen Klangraum durch den Prozessor (130),
- einen Schritt eines Befehligens (207) der Tonausgabevorrichtung (120) durch den Prozessor (130), das Audioausgabesignal zu senden, so dass das Audioausgabesignal für den Patienten von mindestens einer der virtuellen Positionen von virtuellen Tonquellen zu kommen scheint,
- einen Schritt eines Erfassens (208) mindestens einer ersten Bewegung des Patienten durch den Prozessor (130) als Reaktion auf das Senden des Audioausgabesignals,
- einen zweiten Schritt eines periodischen Beziehens (209) mindestens eines Messwerts von der Bewegungserfassungsvorrichtung (180) durch den Prozessor (130) als Reaktion auf die Erfassung der ersten Bewegung des Patienten oder von dem Patienten induzierten Bewegung und bis zur Erfassung mindestens einer zweiten vorbestimmten Bewegung des Patienten oder von dem Patienten induzierten Bewegung, und
- einen Schritt eines Berechnens (210) mindestens eines Scores der räumlichen auditorischen Lokalisation des Patienten zumindest aus dem virtuellen Klangraum und dem Messwert durch den Prozessor (130), wobei der Score der auditorischen Lokalisation die Präzision der Entscheidungsfindung durch den Patienten bei seiner Suche nach der virtuellen Tonquelle, von der das Audioausgabesignal stammt, darstellt und die Berechnung eines Parameters, X3, umfasst, der die Art und Weise bestimmt, auf die der Patient seinen gesamten oder einen Teil seines Körpers bei der Suche nach der virtuellen Tonquelle, von der das Audioausgabesignal stammt, verlagert.

7. Verfahren (200) nach Anspruch 6, wobei der Parameter X3 ein Zähler ist,
wobei das Verfahren (200) weiterhin umfasst:
- einen Schritt eines Haltens des Werts des Zählers auf einem vorbestimmten Wert, während der Patient in eine vorbestimmte Richtung gewandt ist, durch den Prozessor (130) und
- einen Schritt eines Variierens des Werts des Zählers in Abhängigkeit von mindestens einer Richtungsänderung, die sich von der vorbestimmten Richtung unterscheidet, durch den Prozessor (130).

8. Verfahren (200) nach einem der Ansprüche 6 bis 7, weiterhin umfassend:
- einen dritten Schritt eines Beziehens (211) eines biomathematischen Modells, das den Score der räumlichen auditorischen Lokalisation in Abhängigkeit von mindestens einer Klasse eines Vermögens der auditorischen Lokalisation beschreibt, durch den Prozessor (130) und
- einen ersten Schritt eines Verwendens (212) des Scores der räumlichen auditorischen Lokalisation des Patienten als Eingabe in das biomathematische Modell durch den Prozessor (130), um die Klasse des Vermögens der auditorischen Lokalisation, zu der der Patient gehört, zu bestimmen.

9. Verfahren (200) nach Anspruch 8, wobei das biomathematische Modell den Score der auditorischen Lokalisation weiterhin in Abhängigkeit von mindestens einem physiologischen auditorischen Merkmal, das der Morphologie eines Zuhörers eigen ist, beschreibt,
wobei das Verfahren (200) weiterhin umfasst:
- einen vierten Schritt eines Beziehens mindestens eines physiologischen auditorischen Merkmals des Patienten durch den Prozessor (130) und
- einen zweiten Schritt eines Verwendens des Scores der räumlichen auditorischen Lokalisation des Patienten und des physiologischen auditorischen Merkmals des Patienten als Eingaben in das biomathematische Modell durch den Prozessor (130), um die Klasse des Vermögens der auditorischen Lokalisation, zu der der Patient gehört, zu bestimmen.

10. Verfahren (200) nach einem der Ansprüche 6 bis 9, weiterhin umfassend:
- einen fünften Schritt eines Bereitstellens eines beweglichen Steuerhebels (160), dessen Position und Ausrichtung von dem Patienten bestimmt werden und der an den Prozessor (130) gekoppelt ist,
- einen sechsten Schritt eines Bereitstellens mindestens eines zweiten Ausrichtungssensors, der in den Steuerhebel (160) integriert ist, an der Bewegungserfassungsvorrichtung (180), wobei der zweite Ausrichtungssensor dazu konfiguriert ist, mindestens einen Messwert bereitzustellen, der die Ausrichtung in dem Raum des Steuerhebels (160) darstellt, und
- einen siebten Schritt eines Versehens des Steuerhebels (160) mit mindestens einem Steuerknopf, wobei der Steuerknopf dazu konfiguriert ist, ein Signal zu erzeugen, das die Erfassung der zweiten vorbestimmten Bewegung angibt.

## Claims

1. A system (100) for determining a score representative of the spatial auditory localization capability of a patient, hereinafter referred to as spatial auditory localization, the system (100) comprising:
- a visual output device (110) configured to be worn on the patient's head such that movement of the patient's head in a direction of movement causes simultaneous and proportional movement of the visual output device (110) in the direction of movement, the visual output device (110) comprising at least one display unit (111) for displaying a virtual environment to at least one of the patient's eyes,
- a sound output device (120) configured to broadcast at least one output audio signal to at least one of the patient's ears,
- a motion sensing device (180) configured to detect at least one movement of all or part of a patient's body and generate at least one measurement representative of the patient's movement,
- at least one processor (130) coupled to the visual output device (110), the sound output device (120), and the motion sensing device (180), the processor (130) being configured to:
- obtain a virtual auditory space associated with the virtual environment, from a model representative of the acoustic behavior of at least one physical auditory space of the real world or an artificial world, the virtual auditory space being configured to reproduce, in the virtual environment, the acoustic behavior of the physical auditory space of the real world or an artificial world,
- insert and arrange, in the virtual auditory space, a plurality of virtual positions of virtual sound sources, around a virtual position in which the patient observes the virtual environment,
- control the sound output device (120) to broadcast the output audio signal such that, to the patient, the output audio signal appears to be from at least one of the virtual positions of virtual sound sources,
- detect at least a first patient movement or patient-induced movement in response to the broadcast of the output audio signal,
- periodically obtain from the motion sensing device (180) at least one measurement in response to detecting the first patient movement or patient-induced movement and until detecting at least one second predetermined patient movement or patient-induced movement, and
- calculate at least one spatial auditory localization score of the patient, at least from the virtual auditory space and the measurement, the auditory localization score being representative of the accuracy of the patient's decision-making in their search for the virtual sound source from which the output audio signal originates, and comprising calculating a parameter, X3, which determines how the patient moves all or part of their body in search of the virtual sound source from which the output audio signal originates.

2. The system (100) according to claim 1 wherein the parameter, X3, is a counter, and the processor (130) is configured to:
- maintain the counter value at a predetermined value while the patient is moving in a predetermined direction, and
- vary the counter value according to at least one change in direction different from the predetermined direction.

3. The system (100) according to either of claims 1 or 2, wherein the processor (130) is further configured to:
- obtain a biomathematical model that describes the spatial auditory localization score based on at least one auditory localization capability class, and
- use the patient's spatial auditory localization score as an input to the biomathematical model to determine the auditory localization capability class to which the patient belongs.

4. The system (100) according to claim 3, wherein the biomathematical model further describes the auditory localization score based on at least one physiological auditory characteristic specific to a listener's morphology,
the processor (130) being further configured to:
- obtain at least one physiological auditory characteristic of the patient, and
- use the patient's spatial auditory localization score and the patient's physiological auditory characteristic as inputs to the biomathematical model to determine the auditory localization capability class to which the patient belongs.

5. The system (100) according to any one of claims 1 to 4, further comprising a movable joystick (160), the position and orientation of which are determined by the patient, and which is coupled to the processor (130), wherein the motion sensing device (180) comprises at least one second orientation sensor integral with the joystick (160), the second orientation sensor being configured to provide at least one measurement representative of the spatial orientation of the joystick (160), wherein the joystick (160) further comprises at least one control button, the control button being configured to generate a signal indicative of the detection of the second predetermined movement.

6. A method (200) for determining a score representative of the spatial auditory localization capability of a patient, hereinafter referred to as spatial auditory localization, the method (200) comprising:
- a first step (201) of providing a visual output device (110) configured to be worn on the patient's head such that movement of the patient's head in a direction of movement causes simultaneous and proportional movement of the visual output device (110) in the direction of movement, the visual output device (110) comprising at least one display unit (111) for displaying a virtual environment to at least one of the patient's eyes,
- a second step (202) of providing a sound output device (120) configured to broadcast at least one output audio signal to at least one of the patient's ears,
- a third step (203) of providing at least one measurement representative of a predetermined movement of the patient,
- a fourth step (204) of providing at least one processor (130) coupled to the visual output device (110), the sound output device (120) and the motion sensing device (180),
- a first step (205) of obtaining, by the processor (130), a virtual auditory space associated with the virtual environment, from a model representative of the acoustic behavior of at least one physical auditory space of the real world or an artificial world, so that the virtual auditory space reproduces, in the virtual environment, the acoustic behavior of the physical auditory space of the real world or an artificial world,
- a step (206) of inserting and arranging, in the virtual auditory space, by the processor (130), a plurality of virtual positions of virtual sound sources, around a virtual position in which the patient observes the virtual environment,
- a step (207) of controlling, by the processor (130), the sound output device (120) to broadcast the audio output signal such that, to the patient, the audio output signal appears to originate from at least one of the virtual positions of virtual sound sources,
- a step (208) of detecting, by the processor (130), at least a first patient movement in response to the broadcast of the output audio signal,
- a second step (209) of periodically obtaining from the motion sensing device (180) by the processor (130) at least one measurement in response to detecting the first patient movement or patient-induced movement and
until detecting at least one second predetermined patient movement or patient-induced movement, and
- a step (210) of calculating, by the processor (130), at least one spatial auditory localization score of the patient, at least from the virtual auditory space and the measurement, the auditory localization score being representative of the accuracy of the patient's decision-making in their search for the virtual sound source from which the audio output signal originates, and comprising the calculation of a parameter, X3, which determines how the patient moves all or part of their body in search of the virtual sound source from which the audio output signal originates.

7. The method (200) according to claim 6, wherein the parameter,
X3, is a counter,
the method (200) further comprising:
- a step of maintaining the counter value by the processor (130) at a predetermined value while the patient is moving in a predetermined direction, and
- a step of varying the counter value by the processor (130) according to at least one change in direction different from the predetermined direction.

8. The method (200) according to either one of claims 6 to 7, further comprising:
- a third step (211) of obtaining, by the processor (130), a biomathematical model that describes the spatial auditory localization score based on at least one auditory localization capability class, and
- a first step (212) of using, by the processor (130), the patient's spatial auditory localization score as an input to the biomathematical model to determine the auditory localization capability class to which the patient belongs.

9. The method (200) according to claim 8, wherein the biomathematical model further describes the auditory localization score based on at least one physiological auditory characteristic specific to a listener's morphology,
the method (200) further comprising:
- a fourth step of obtaining, by the processor (130), at least one physiological auditory characteristic of the patient, and
- a second step of using, by the processor (130), the patient's spatial auditory localization score and the patient's physiological auditory characteristic as inputs to the biomathematical model to determine the auditory localization capability class to which the patient belongs.

10. The method (200) according to any one of claims 6 to 9, further comprising:
- a fifth step of providing a movable joystick (160), the position and orientation of which are determined by the patient, and which is coupled to the processor (130),
- a sixth step of providing, to the motion sensing device (180), at least one second orientation sensor integrated with the joystick (160), the second orientation sensor being configured to provide at least one measurement representative of the spatial orientation of the joystick (160), and
- a seventh step of providing, to the joystick (160), at least one control button the control button being configured to generate a signal indicative of the detection of the second predetermined movement.
